# EUROPEAN PATENT SPECIFICATION

(11) **EP 3 498 112 B1**
(45) Date of publication and mention of the grant of the patent: **01.07.2020**
(21) Application number: 16913419.4
(22) Date of filing: 12.12.2016
(51) Int. Cl.: A24F 47/00, A61M 15/06, A61M 11/00, A61M 11/04

(54) **ELECTRONIC CIGARETTE**
ELEKTRONISCHE ZIGARETTE
CIGARETTE ÉLECTRONIQUE

(30) Priority: 15.08.2016 CN 201610667446
(43) Date of publication of application: 19.06.2019
(73) Proprietor: Changzhou Jwei Intelligent Technology Co., Ltd., Changzhou, Jiangsu 213125 (CN); Joyetech Europe Holding GmbH, 6303 Zug (CH)
(72) Inventor: QIU, Weihua, Jiangsu 213125 (CN)
(74) Representative: Zaboliene, Reda
(86) International application number: PCT/CN2016/109532
(87) International publication number: WO 2018/032667

(56) References cited:
- EP-A1- 2 047 880
- WO-A1-2017/108394
- CN-A- 104 770 898
- CN-A- 104 872 820
- CN-A- 104 872 820
- CN-A- 106 108 121
- CN-U- 204 146 331
- CN-U- 204 377 920
- CN-U- 204 519 371
- CN-U- 205 947 127
- GB-A- 2 524 856
- US-A1- 2009 151 717
- US-A1- 2015 122 278

## Description

### TECHNICAL FIELD

The present disclosure relates to the field of electronic cigarette technology, and in particular, to an electronic cigarette with less dry burning and liquid leakage problems.

### BACKGROUND

Most electronic cigarettes on the market use fiber rope or composite cotton to achieve liquid guiding. When the user uses the electronic cigarette, it is easy to burn fiber rope or cotton due to less e-cigarette liquid infiltrated on the liquid guiding member. When dry burning, the fiber rope or composite cotton is directly heated by the heating wire to emit burnt smoke, which is unpleasant and harmful to the human body, and also affects the taste of smoking. When there is a large amount of e-cigarette liquid infiltrated on the liquid guiding element and it is too much to be consumed, there is a risk of liquid leakage. Leakage and dry burning are common problems of current electronic cigarettes.

CN104872820A discloses an atomizing device including a shell. A liquid storage barrel used for containing liquid is arranged in the shell, a tubular or needle-shaped liquid outlet is arranged at one end of the liquid storage barrel, a piston capable of sliding along the inner wall of the liquid storage barrel is arranged at the other end of the liquid storage barrel and driven by a driving device to slide towards the liquid outlet, and an electric heating device is arranged at the liquid outlet.

EP2047880A1 discloses an aerosol aspirator. The aerosol aspirator has a casing with a mouthpiece and an outside air inlet, a generation passage extending from the outside air inlet to the mouthpiece, a syringe pump arranged within the casing to deliver a solution to a distributing position in the generation passage in a fixed amount each time it is activated, and a tubular heater disposed downstream of the distributing position and forming a part of the generation passage.

GB2524856A discloses a pulmonary delivery apparatus comprising: a first chamber adapted to thermally vaporise a quantity of a first liquid to form a relatively warm first vapour and a second chamber adapted to atomize a quantity of a second liquid without heating of the second liquid to form a mist of a relatively cold second vapour, and an outlet via which, in use, a user can inhale a mixture of the first and second vapours. The device may resemble a cigarette, pipe or cigar.

CN104770898A discloses an electronic atomization device, which includes an atomizer main body with both an atomization bin and an electric conductor arranged therein, a second fixing sleeve pipe, a motor assembly, a slider, and a heating component electrically connected with the electric conductor. The motor assembly comprise a motor, a gearbox, a switching circuit control panel, a control circuit board and a first fixing sleeve pipe. The slider is provided with a cavity for accommodating the heating component. A screw used for driving the slider to slide is arranged on one end surface of the first fixing sleeve pipe and fixedly connected with the gearbox. One end of the control circuit board is electrically connected with the power supply, and the other end of the control circuit board is electrically connected with the switching circuit control panel. The switching circuit control panel is electrically connected with the electric conductor.

WO2017108394A1 discloses an aerosol-generating system including a liquid storage portion for storing liquid aerosol-forming substrate, wherein the liquid storage portion includes a movable wall and an outlet, a vaporiser including a heating element having a structure defining an open-ended internal passage for heating deposited liquid aerosol-forming substrate, and a pump configured for delivering liquid aerosol-forming substrate from the outlet of the liquid storage portion to the internal passage of the heating element. The pump includes a micro stepper motor with a drive shaft that is configured to rotate for a predetermined amount upon performing one step of the micro stepper motor, a piston connected to the movable wall, and a lead screw connecting the drive shaft to the piston and configured to translate a rotation of the drive shaft into an axial movement of the piston and a corresponding axial movement of the movable wall.

### SUMMARY

To overcome the above problems, the present disclosure provides an electronic cigarette with less dry burning and liquid leakage problems.

The technical solution adopted by the present disclosure to address the above problems is as defined in claim 1 and its appended claims.

An electronic cigarette includes a liquid storage device and an atomizing device. The liquid storage device includes a liquid storage chamber. The atomizing device includes an atomizing chamber. The electronic cigarette further includes a controller and a driving mechanism. The driving mechanism is electrically connected to and controlled by the controller. When the controller controls a liquid intake of the electronic cigarette, the controller controls the driving mechanism to drive e-cigarette liquid in the liquid storage chamber to enter the atomizing chamber.

In one embodiment, after the use of the electronic cigarette, the controller controls the driving mechanism to drive residual e-cigarette liquid in the atomizing chamber to return back to the liquid storage chamber.

In one embodiment, the electronic cigarette further includes a guiding groove in communication with the liquid storage chamber. The atomizing device further includes a liquid inlet. The atomizing chamber is in communication with the liquid storage chamber through the liquid inlet. The driving mechanism includes a driving motor, a screw rod and a slider. The screw rod and the slider are disposed in the guiding groove. The driving motor is disposed at a side of the guiding groove away from the liquid storage chamber. The screw rod is connected to the driving motor, the slider is connected to the screw rod. The screw rod driven by the driving motor drives the slider to move in a direction close to the liquid storage chamber or in a direction away from the liquid storage chamber.

In one embodiment, the slider is slidably connected to the screw rod; when the driving motor rotates, the screw rod is driven to rotate and drives the slider to slide in the direction close to the liquid storage chamber or to slide in the direction away from the liquid storage chamber.

In one embodiment, the slider is fixed at one end of the screw rod near the liquid storage chamber. The screw rod is driven by the driving motor to make a telescopic motion and drives the slider to move in the direction close to the liquid storage chamber or in the direction away from the liquid storage chamber in the guiding groove.

In one embodiment, the controller includes a control switch and a control board. The control switch is signally connected to the control board. The driving mechanism is electrically connected to and controlled by the control board.

In one embodiment, the controller further includes an input device, the input device is signally connected to the control board. The input device is configured for inputting desired operating temperature/operating power/operating voltage of the atomizing device to the control board.

In one embodiment, the control switch is a key switch or a touch switch. The control board is preset with a corresponding relationship between the rotational speed of the driving motor and the operating temperature/operating power/operating voltage of the atomizing device.

In one embodiment, the control switch is an airflow sensor configured to detect the flow velocity of the airflow when the user is smoking. The control board is preset with a corresponding relationship between the rotational speed of the driving motor and the flow velocity of the airflow.

In one embodiment, the electronic cigarette further includes an airflow detection channel, and the airflow sensor is located in the airflow detection channel.

In one embodiment, the liquid storage device further includes a liquid inlet tube, a first end of the liquid inlet tube is in communication with the atomizing chamber, and a second end of the liquid inlet tube is in communication with the liquid storage chamber. The controller controls the driving mechanism to drive the e-cigarette liquid in the liquid storage chamber into the atomizing chamber through the liquid inlet tube.

In one embodiment, the driving mechanism includes a piston, a connecting member and a driving motor. The piston is located in the liquid storage chamber. The piston divides the liquid storage chamber into a hollow chamber and a liquid chamber, the liquid chamber is in communication with the atomizing chamber through the liquid inlet tube. The connecting member is connected between the piston and the driving motor. The connecting member drives the piston to move towards the liquid chamber under the action of the driving motor.

In one embodiment, the second end of liquid inlet tube passes through the hollow chamber and the piston in sequence, and the second end of the liquid inlet tube is inserted into the liquid chamber.

In one embodiment, the liquid storage device further includes a connecting tube disposed between the liquid chamber and the liquid inlet tube. The liquid chamber is in communication with the liquid inlet tube through the connecting tube.

In one embodiment, the connecting member includes a screw rod, a slider, a guide rail and a connecting rod. The screw rod is connected to the driving motor, the screw rod extends through the slider. The slider is slidably connected to the guide rail, one end of the connecting rod is fixedly connected to the slider, and the other end of the connecting rod is fixedly connected to the piston.

In one embodiment, the electronic cigarette further includes a liquid level detection device. The liquid level detection device is disposed in the liquid storage chamber. The electronic cigarette further includes a display screen. The liquid level detection device is electrically connected to the display screen.

In one embodiment, the electronic cigarette further includes a housing. The liquid storage device is located in the housing. An injection hole is defined at a bottom of the liquid storage chamber. An opening is provided at the housing corresponding to the injection hole. The electronic cigarette further includes a liquid injection assembly for covering the opening.

In one embodiment, when the injection hole is in communication with a liquid bottle, the drive motor is controlled by the controller to rotate reversely, leading the connecting member to drive the piston to move towards the hollow chamber to achieve automatic liquid injection.

In one embodiment, the liquid injection assembly includes a mounting member, a cover and a telescopic liquid injection tube. The telescopic liquid injection tube is fixedly connected to the mounting member. The cover is rotatably connected to the mounting member. The mounting member is connected to the housing. An upper port of the telescopic liquid injection tube is sealing connected to the opening. The cover has an open state and a closed state. When the cover is in the closed state, a lower port of the telescopic liquid injection tube is sealing abutted against the cover.

In one embodiment, a convex ring protrudes outwards from the housing, a groove is defined in the mounting member for matching with the convex ring, and the housing is detachably connected to the mounting member through the convex ring and the groove.

In one embodiment, a center of the mounting member has a through hole, an upper end of the telescopic liquid injection tube is connected to the mounting member, the upper port of the telescopic liquid injection tube communicates with the liquid storage chamber through the through hole.

In one embodiment, the telescopic liquid injection tube includes a first tube and a plurality of telescopic tubes, the first tube is fixedly connected to the mounting member. Every two adjacent telescopic tubes are nested and connected, such that a lower telescopic tube is not separated from an upper telescopic tube.

In one embodiment, a one-way conducting member is located in the liquid inlet tube. The one-way conducting member prevents the e-cigarette liquid in the atomizing chamber from flowing back into the liquid storage chamber when the e-cigarette liquid is injected into the liquid storage chamber through the liquid injection assembly.

In one embodiment, the controller includes a control board, a liquid inlet control switch and/or a liquid injection control switch. The liquid inlet control switch and/or the liquid injection control switch are signally connected to the control board. The driving mechanism is controlled by the control board. When the liquid inlet control switch or the liquid injection control switch is turned on, the control board is triggered and the control board controls the movement of the driving mechanism.

In one embodiment, the liquid injection control switch and the liquid inlet control switch are the same switch.

In one embodiment, the controller further includes a liquid injection amount control switch and/or a liquid intake amount control switch. The liquid injection amount control switch and/or liquid intake amount control switch are signally connected to the control board.

In one embodiment, the liquid injection amount control switch is a mechanical type. The liquid injection amount control switch includes a plurality of gear positions, and each gear position corresponds to a liquid injection amount each time. Or, the liquid injection amount control switch is a touch screen type, and the liquid injection amount control switch of the touch screen type is configured to set the value of the liquid injection amount.

In one embodiment, the liquid intake amount control switch is a mechanical type. The liquid intake amount control switch includes a plurality of gear positions, and each gear position corresponds to a liquid intake amount each time. Or, the liquid intake amount control switch is a touch screen type, and the liquid intake amount control switch of the touch screen type is configured to set the value of the liquid intake amount.

In one embodiment, the controller further includes an atomizing control switch, the atomizing control switch and the liquid inlet control switch are controlled separately or controlled in linkage.

In one embodiment, the liquid inlet control switch and the atomizing control switch are the same switch.

Compared with the prior art, the beneficial effects of the present disclosure are:

In the electronic cigarette of the present disclosure, the controller controls the driving mechanism to drive the e-cigarette liquid in the liquid storage chamber to enter into the atomizing device, thereby realizing the control of the amount of the e-cigarette liquid in the atomizing device. That is, during smoking of the user, the electronic cigarette can always provide an appropriate amount of e-cigarette liquid in the atomizing device, and there is no dry burning or liquid leakage caused by insufficient atomization.

### BRIEF DESCRIPTION OF THE DRAWINGS

FIG. 1 is a cross-sectional view of an electronic cigarette according to a first embodiment of the present disclosure;
FIG. 2 is a block diagram of the electronic cigarette of the first embodiment;
FIG. 3 is a cross-sectional view of an electronic cigarette according to a second embodiment of the present disclosure;
FIG. 4 is a block diagram of the electronic cigarette of the second embodiment;
FIG. 5 is a cross-sectional view of an electronic cigarette according to a third embodiment of the present disclosure, in which the driving mechanism is simplified;
FIG. 6 is a right side view of the electronic cigarette shown in FIG. 5;
FIG. 7 is a top view of the electronic cigarette shown in FIG. 5;
FIG. 8 is a schematic view of the driving mechanism and the liquid storage device according to one example of the third embodiment;
FIG. 9 is a schematic view of the driving mechanism and the liquid storage device according to another example of the third embodiment;
FIG. 10 is a schematic view of the liquid injection device of the third embodiment.

The following table lists various components and reference numerals thereof.

| | |
|---|---|
| atomizing device 110, 210 | atomizing chamber 111, 211 |
| liquid storage chamber 121, 220 | vent pipe 122 |
| mouthpiece 131 | mouthpiece connector 132 |
| air intake hole 133 | controller 140 |
| airflow sensor 141b | screw rod 152, 2441 |
| driving motor 151, 242 | hollow chamber 220b |
| housing 170, 250 | first end 222a |
| liquid chamber 220a | piston 240 |
| liquid inlet tube 222 | guide rail 2443 |
| control board 142, 232 | battery 160, 252 |
| slider 153, 2442 | telescopic liquid injection tube 264 |
| observation window 2501 | liquid storage device 120 |
| cover 262 | mouthpiece assembly 130 |
| connecting tube 270 | airflow detection channel 1322 |
| liquid inlet 112 | key switch 141a |
| air outlet pipe 123 | driving mechanism 150 |
| guiding groove 1321 | first tube 2641 |
| control switch 141 | injection hole 2202 |
| input device 143 | liquid inlet control switch 230 |
| convex ring 2201 | groove 2601 |
| second end 222b | telescopic tube 2642 |
| connecting rod 2445 | mounting member 260 |

### DETAILED DESCRIPTION OF PREFERRED EMBODIMENTS

The present disclosure will now be further described with reference to the accompanying drawings and embodiments.

### First embodiment

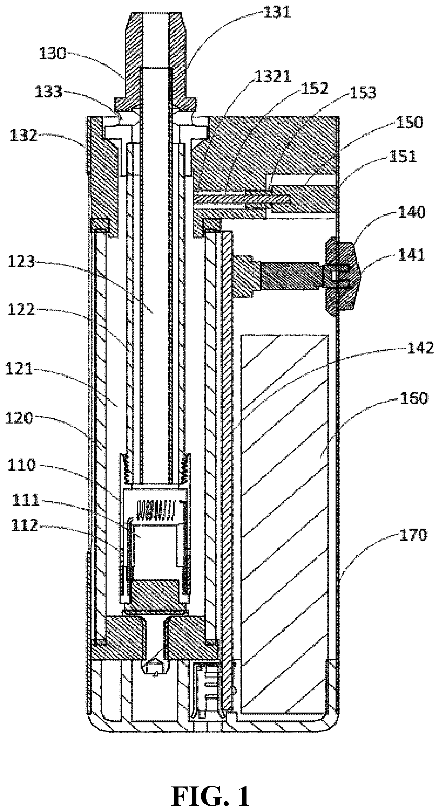

Referring to FIG. 1, an electronic cigarette in the first embodiment includes a liquid storage device 120, an atomizing device 110, a controller 140, and a driving mechanism 150. The liquid storage device 120 includes a liquid storage chamber 121. The atomizing device 110 is received in the liquid storage chamber 121. The driving mechanism 150 is electrically connected to and controlled by the controller 140. The controller 140 controls the amount of the e-cigarette liquid in the liquid storage chamber 121 to enter and exit the atomizing device 110. Specifically, the controller 140 can control the amount of e-cigarette liquid driven by the driving mechanism 150 into and out of the atomizing device 110. As long as the controller 140 controls the entry and exit of e-cigarette liquid of the electronic cigarette, the driving mechanism 150 drives the e-cigarette liquid in the liquid storage chamber 121 to enter and exit the atomizing device 110, thereby realizing the control of the amount of the e-cigarette liquid in the atomizing device 110. Thereby, when the user smokes, there is an appropriate amount of e-cigarette liquid in the atomizing device 110, there is no dry burning and liquid leakage due to insufficient atomization. After smoking, the e-cigarette liquid in the atomizing device 110 returns to the liquid storage chamber 121, thereby reducing the possibility of leakage of residual e-cigarette liquid, and ensuring the taste of the user when smoking again.

The atomizing device 110 includes an atomizing chamber 111 and a liquid inlet 112 in communication with the atomizing chamber 111. The atomizing device 110 is in communication with the liquid storage chamber 121 through the liquid inlet 112. That is, when the electronic cigarette is in use, the e-cigarette liquid enters the atomizing chamber 111 of the atomizing device 110 from the liquid storage chamber 121 through the liquid inlet 112 under the action of the driving mechanism 150. When not in use, the e-cigarette liquid can also return to the liquid storage chamber 121 from the atomizing chamber 111 under the action of the driving mechanism 150.

The electronic cigarette further includes a mouthpiece assembly 130. The mouthpiece assembly 130 includes a mouthpiece connector 132 and a mouthpiece 131 disposed on the mouthpiece connector 132. The mouthpiece connector 132 is disposed on the liquid storage device 120. A guiding groove 1321 in communication with the liquid storage chamber 121 is defined in the mouthpiece connector 132. The mouthpiece connector 132 is further provided with an air intake hole 133 for air to enter the atomizing chamber 111.

A vent pipe 122 is disposed in the liquid storage chamber 121. The vent pipe 122 has a cylindrical structure, one end of the vent pipe 122 is in communication with the atomizing device 110, the other end of the vent pipe 122 is in communication with the air intake hole 133. An air outlet pipe 123 is disposed in the vent pipe 122. One end of the air outlet pipe 123 is in communication with the atomizing device 110, and the other end of the air outlet pipe 123 is in communication with the mouthpiece 131. The outside air enters the gap between the vent pipe 122 and the air outlet pipe 123 through the air intake hole 133, flows to the top end of the atomizing device 110 and mixes with the smoke formed by the atomization, and then flows out through the air outlet pipe 123 and is inhaled by the user at the mouthpiece 131.

The driving mechanism 150 includes a driving motor 151, a screw rod 152 and a slider 153. The screw rod 152 is connected to the driving motor 151, the slider 153 is slidably connected to the screw rod 152. Thus, the screw rod 152 is driven to rotate when the driving motor 151 rotates, thereby driving the slider 153 to move along the screw rod 152. The screw rod 152 and the slider 153 are disposed in the guiding groove 1321, the driving motor 151 is disposed at the side of the guiding groove 1321 away from the liquid storage chamber 121. The slider 153 can reciprocate in the guiding groove 1321 under the driving of the screw rod 152 to adjust the pressure in the liquid storage chamber 121. That is, when the slider 153 moves toward the liquid storage chamber 121, the pressure inside of the liquid storage chamber 121 increases and the amount of e-cigarette liquid in the liquid storage chamber 121 entering the atomizing chamber 111 increases. When the slider 153 moves away from the liquid storage chamber 121, the pressure in the liquid storage chamber 121 decreases, and the e-cigarette liquid in the atomizing chamber 111 returns to the liquid storage chamber 121. The moving speed of the slider 153 also affects the flow rate of the e-cigarette liquid. Taking the slider 153 moving toward the liquid storage chamber 121 as an example, the slider 153 moves fast, and the flow rate of the e-cigarette liquid into the atomizing chamber 111 is high, and conversely, the flow rate of the e-cigarette liquid into the atomizing chamber 111 is low. In addition, by adopting the screw rod, precise quantitative e-cigarette liquid feeding can be realized. Specifically, when the screw rod 152 is rotated one turn, the slider 153 can move linearly by a certain distance, and quantitative supply of e-cigarette liquid can be achieved. It can be understood that, the driving motor 151 can adopt any form of motor, when the driving motor 151 is a stepping motor or a servo motor, the movement of the slider 153 can be controlled more precisely.

It is understood that, in an embodiment not shown, the cross section of the guiding groove 1321 is circular, the slider 153 is disposed at one end of the screw rod 152 near the liquid storage chamber 121. The screw rod 152 is driven by the driving motor 151 to make a telescopic motion and drives the slider 153 to move in a direction toward or away from the liquid storage chamber 121 in the guiding groove 1321. It can be understood that, the driving motor 151 can be replaced with a cylinder. It can be understood that, the cross section of the guiding groove 1321 can also be a non-circular structure such as a rectangle, an ellipse or a diamond. In order to ensure a smoother movement of the slider 153 and the screw rod 152, the slider 153 is also selected to have a shape matching the guiding groove 1321, such as a rectangle, an ellipse, a diamond, or the like.

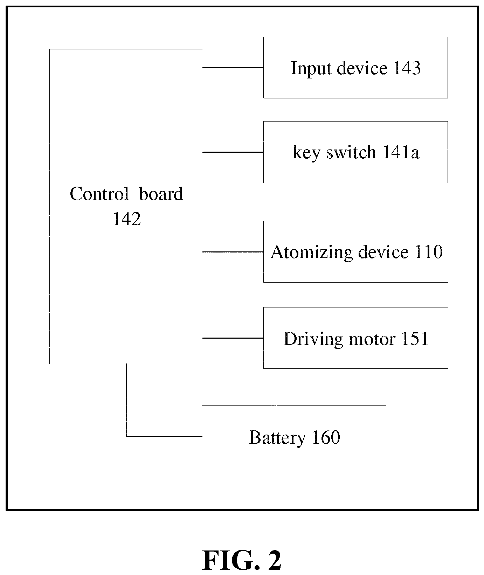

Referring to FIG. 2, in the embodiment, the controller 140 includes a control switch 141, a control board 142, and an input device 143. The control switch 141 is a key switch 141a, the input device 143 is a touch display screen, the key switch 141a and the input device 143 are separately and signally connected to the control board 142. The input device 143 is configured for inputting data of the atomizing device 110 into the control board 142, such as operating temperature, operating power and operating voltage, etc. The control board 142 is preset with a corresponding relationship between the rotational speed of the driving motor 151 and the operating temperature/operating power/operating voltage of the atomizing device 110. The driving mechanism 150 is electrically connected to and controlled by the control board 142, when the key switch 141a is turned on, the control board 142 is triggered and controls the movement of the driving mechanism 150. The user inputs a desired operating temperature/operating power/operating voltage into the control board 142 through the input device 143. The control board 142 controls the rotation speed of the driving motor 151 according to the input data and the preset corresponding relationship between the rotation speed of the driving motor 151 and the operating temperature/operating power/operating voltage of the atomizing device 110, thereby realizing the control of the liquid intake amount.

It can be understood that, the control switch 141 can control the liquid storage chamber 121 to feed e-cigarette liquid into the atomizing chamber 111, and can also control the e-cigarette liquid in the atomizing chamber 111 to be sucked back into the liquid storage chamber 121, and can also control the turning on and off of the atomizing device 110. The input device 143 can be a touch display, the control switch 141 can be a touch switch.

The electronic cigarette further includes a battery 160 and a housing 170. The liquid storage device 120, the atomizing device 110, the driving mechanism 150 and the battery 160 are all received in the housing 170. The atomizing device 110, the controller 140, and the driving mechanism 150 are electrically connected to the battery 160, respectively. To facilitate the observation of the e-cigarette liquid in the liquid storage chamber 121, the material of the peripheral wall of the liquid storage chamber 121 may be made of a transparent material such as glass or transparent resin. An observation window (not shown) is formed on the housing 170, the amount of the e-cigarette liquid in the liquid storage chamber 121 can be visually observed through the observation window.

In the embodiment, when the electronic cigarette is in use, the user inputs a desired operating temperature/operating power/operating voltage through the touch display screen, and the touch display screen transmits the input data to the control board 142. The control board 142 is triggered by the user through the key switch 141a, the control board 142 matches the input data received from the touch display screen with the preset corresponding relationship between the rotational speed of the driving motor 151 and the operating temperature/operating power/operating voltage of the atomizing device 110, and controls the driving motor 151 to rotate. In the initial state, the slider 153 stays on a side of the guiding groove 1321 away from the liquid storage chamber 121. When the desired operating temperature/operating power/operating voltage is large, the atomizing device 110 has a large atomization rate, the driving motor 151 rotates fast, and the slider 153 moves rapidly toward the liquid storage chamber 121, the e-cigarette liquid in the liquid storage chamber 121 rapidly enters the atomizing chamber 111, to satisfy the requirement of the atomizing device 110 for the amount of e-cigarette liquid and avoid dry burning. When the desired operating temperature/operating power/operating voltage is small, the atomization rate of the atomizing device 110 is small, the driving motor 151 rotates at a low speed or does not rotate, the slider 153 moves slowly or does not move in a direction towards the liquid storage chamber 121, the e-cigarette liquid in the liquid storage chamber 121 slowly enters the atomizing chamber 111, to satisfy the requirement of the atomizing device 110 for the amount of e-cigarette liquid and avoid excessive e-cigarette liquid and insufficient atomization. After the use of the electronic cigarette, the driving motor 151 drives the slider 153 to move in a direction away from the liquid storage chamber 121, that is, the initial state is restored. At this time, the pressure in the liquid storage chamber 121 is reduced, the excessive e-cigarette liquid in the atomizing chamber 111 returns back to the liquid storage chamber 121 under the action of air pressure, the residual of the e-cigarette liquid in the atomizing chamber 111 is avoided. This can reduce the possibility of leakage of e-cigarette liquid when the electronic cigarette is not used, and ensure that the user does not inhale the residual e-cigarette liquid when the user smokes again, thereby ensuring the taste of smoking.

### Second embodiment

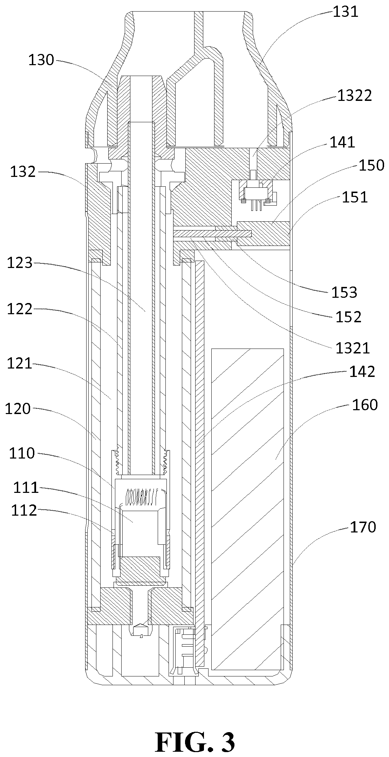

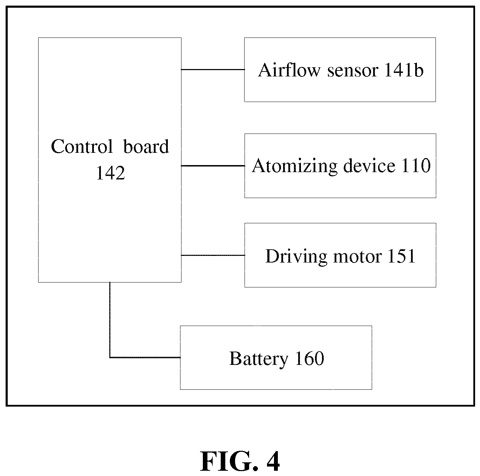

Please refer to FIG. 3 and FIG. 4, the difference between the second embodiment and the first embodiment is as follows. In the embodiment, the control switch 141 is an airflow sensor 141b. An airflow detection channel 1322 in communication with the mouthpiece 131 is defined in the mouthpiece connector 132, the airflow sensor 141b is located in the airflow detection channel 1322. The airflow sensor 141b can detect the velocity of the airflow in the airflow detection channel 1322 when the user smokes. A corresponding relationship between the rotational speed of the driving motor 151 and the flow velocity of the airflow is preset in the control board 142. It should be noted that, when the airflow sensor 141b detects the airflow, the atomizing device 110 is triggered, the control board 232 controls the rotational speed of the driving motor 151 based on the flow velocity detected by the airflow sensor 141b to control the liquid intake amount.

It can be understood that, as long as the detection of the flow velocity of the airflow in the electronic cigarette can be achieved, the airflow sensor 141b can also be disposed at other positions of the electronic cigarette, such as in the gap between the vent pipe 122 and the air outlet pipe 123, or in the air intake hole 133 of the electronic cigarette.

In this embodiment, when the electronic cigarette is in use, the user inhales the electronic cigarette, the airflow sensor 141b detects the airflow in the airflow detection channel 1322. While the atomizing device 110 is turned on, the data of the airflow velocity is sent to the control board 142 and the control board 142 is triggered. Then, the control board 142 matches the data received from the airflow sensor 141b with the preset relationship between the rotational speed of the driving motor 151 and the flow velocity of the airflow, and controls the driving motor 151 to rotate. In the initial state, the slider 153 stays on the side of the guiding groove 1321 away from the liquid storage chamber 121. When the detected airflow rate is large, the atomizing device 110 generates a large amount of smoke, the driving motor 151 rotates fast to drive the slider 153 to move rapidly toward the liquid storage chamber 121, the e-cigarette liquid in the liquid storage chamber 121 rapidly enters the atomizing chamber 111, to satisfy the demand of the atomizing device 110 for the amount of e-cigarette liquid and avoid the occurrence of dry burning. When the detected airflow rate is small, the atomizing device 110 generates a small amount of smoke, the driving motor 151 has a low rotation speed or does not rotate, so that the slider 153 moves slowly or does not move in a direction towards the liquid storage chamber 121, and the e-cigarette liquid in the liquid storage chamber 121 slowly enters the atomizing chamber 111, to satisfy the requirement of the atomizing device 110 for the amount of e-cigarette liquid and avoid excessive e-cigarette liquid and insufficient atomization. After the use of the electronic cigarette, the driving motor 151 drives the slider 153 to move in a direction away from the liquid storage chamber 121, that is, the initial state is restored. At this time, the pressure in the liquid storage chamber 121 is reduced, the excessive e-cigarette liquid in the atomizing chamber 111 returns back to the liquid storage chamber 121 under the action of the air pressure, thereby avoiding the residual of the e-cigarette liquid in the atomizing chamber 111. This can reduce the possibility of leakage of e-cigarette liquid when the electronic cigarette is not used, and ensure that the user does not inhale the residual e-cigarette liquid when the user smokes again, thereby ensuring the taste of smoking.

### Third embodiment

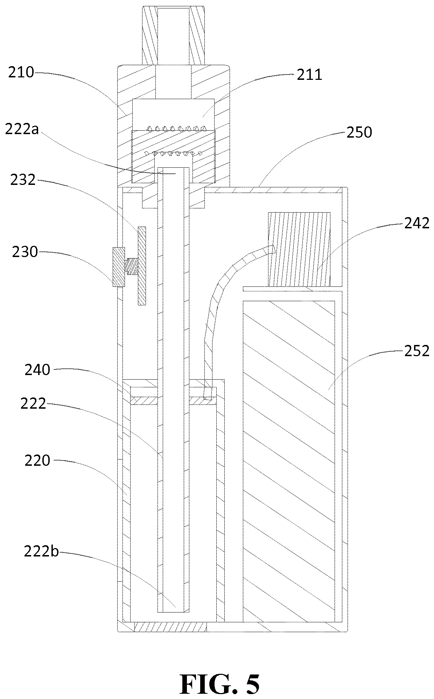

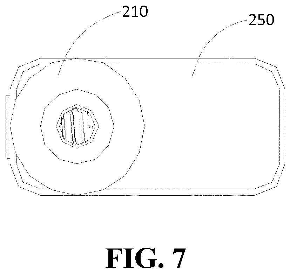

Referring to FIG. 5 to FIG. 7, in this embodiment, the electronic cigarette includes an atomizing device 210 and a liquid storage device. The atomizing device 210 includes an atomizing chamber 211. The electronic cigarette further includes a controller and a driving mechanism. The controller controls the liquid intake of the electronic cigarette. The liquid storage device includes a liquid inlet tube 222 and a liquid storage chamber 220. A first end 222a of the liquid inlet tube 222 is in communication with the atomizing chamber 211, a second end 222b of the liquid inlet tube 222 is in communication with the liquid storage chamber 220. The driving mechanism is electrically connected to and controlled by the controller. When the controller controls the liquid intake of the electronic cigarette, the controller controls the driving mechanism to drive the e-cigarette liquid in the liquid storage chamber 220 to enter the atomizing chamber 211 through the liquid inlet tube 222. As long as the controller controls the liquid intake of the electronic cigarette, the driving mechanism drives the e-cigarette liquid in the liquid storage chamber 220 to enter the atomizing chamber 211 through the liquid inlet tube 222, thereby ensuring that there is an appropriate amount of e-cigarette liquid in the atomizing chamber 211, and dry burning does not occur. Therefore, the present disclosure can effectively solve the dry burning problem of the electronic cigarette. At the same time, the amount of e-cigarette liquid entering the atomizing chamber 211 through the liquid inlet tube 222 is controlled by the driving mechanism, the movement of the driving mechanism is controlled by the controller. Thus, the amount of the e-cigarette liquid entering the atomizing chamber 211 through the liquid inlet tube 222 can be controlled by the driving mechanism, there is no risk of liquid leakage due to infiltration of more e-cigarette liquid on the liquid guiding element than consumption in the prior art. Therefore, the present disclosure can also solve the problem of the leakage of e-cigarette liquid.

The controller includes a control board 232 and a liquid inlet control switch 230 and/or a liquid injection control switch. The liquid inlet control switch 230 and/or the liquid injection control switch are signally connected to the control board 232, the driving mechanism is controlled by the control board 232. When the liquid inlet control switch 230 or the liquid injection control switch is turned on, the control board 232 is triggered, and the control board 232 controls the movement of the driving mechanism. It should be noted that, in one embodiment, when the controller includes the liquid inlet control switch 230 and the control board 232, that is, the liquid injection control switch is not included, the liquid inlet control switch 230 is signally connected to the control board 232, the driving mechanism is controlled by the control board 232. When the liquid inlet control switch 230 is turned on, the control board 232 is triggered and the control board 232 controls the movement of the driving mechanism.

In another embodiment, when the controller includes a liquid injection control switch and a control board 232, that is, the liquid inlet control switch 230 is not included, the liquid injection control switch is signally connected to the control board 232, the driving mechanism is controlled by the control board 232. When the liquid injection control switch is turned on, the control board 232 is triggered, and the control board 232 controls the movement of the driving mechanism.

It can be understood that, when the controller includes the control board 232, the liquid inlet control switch 230 and the liquid injection control switch, the liquid inlet control switch 230 and the liquid injection control switch are signally connected to the control board 232, the driving mechanism is controlled by the control board 232. When the liquid inlet control switch 230 or the liquid injection control switch is turned on, the control board 232 is triggered, and the control board 232 controls the movement of the driving mechanism.

Referring to FIG. 5, in the embodiment, the controller includes a liquid inlet control switch 230 and a control board 232. The liquid inlet control switch 230 is signally connected to the control board 232, the driving mechanism is controlled by the control board 232. When the liquid inlet control switch 230 is turned on, the control board 232 is triggered. Since the driving mechanism is controlled by the control board 232, the triggered control board 232 can control the movement of the driving mechanism. It can be understood that the control board 232 is provided with a chip, the amount of movement of the driving mechanism can be controlled by the chip, thus the amount of the e-cigarette liquid entering the atomizing chamber 211 at each time can be set to realize the quantitative liquid intake. It can be understood that, the liquid inlet control switch can be a mechanical physical button or a virtual button on the touch screen. For example, referring to FIG. 5, in the embodiment, the liquid inlet control switch is a mechanical physical button. In one embodiment not shown, the liquid inlet control switch can share the same switch as the switch that controls the atomization of the electronic cigarette, so that it is not necessary to separately set a liquid inlet control switch. When the atomization of the electronic cigarette is turned on, the intake of e-cigarette liquid can be automatically started, that is, as long as the user is smoking the electronic cigarette, it is ensured that the atomizing chamber 211 has an appropriate amount of the e-cigarette liquid, so that the dry burning problem of the electronic cigarette can be completely avoided. In order to facilitate the control of the amount of liquid intake, the controller further includes a liquid intake amount control switch (not shown). The liquid intake amount control switch is signally connected to the control board 232 for transmitting the information of the liquid intake amount control switch to the control board 232 for controlling the amount of e-cigarette liquid to be fed each time. The liquid intake amount control switch may be a mechanical physical button or a virtual button on the touch screen. For example, when the electronic cigarette adopts a mechanical liquid intake amount control switch, a plurality of gear positions can be set, and each gear position corresponds to the amount of liquid inlet each time. When the electronic cigarette adopts the liquid intake amount control switch of a touch screen type, the value of specific amount of liquid intake can be set on the touch screen with more flexibility.

Further, the controller further includes an atomizing control switch (not shown). When the atomizing control switch is pressed, the electronic cigarette turns on the atomization, and the electronic cigarette can be smoked at this time. It can be understood that, the liquid inlet control switch 230 and the atomizing control switch can be controlled separately or controlled in linkage. When the liquid inlet control switch 230 is controlled in linkage with the atomizing control switch, that is, when the atomizing control switch is turned on, the liquid inlet control switch is automatically turned on. That is, when the user smokes the electronic cigarette, the liquid intake of the electronic cigarette automatically starts, so that the dry burning problem can be completely avoided. It can be understood that, when the liquid inlet control switch 230 and the atomizing control switch are separately controlled, the liquid inlet control switch 230 can be first pressed to make an appropriate amount of e-cigarette liquid in the atomizing chamber 211, and then the atomizing control switch is pressed for smoking.

It should be noted that, when the driving mechanism is controlled by the control board 232, the working of the driving mechanism and the control board 232 can be at almost the same time or can be delayed by a set time. For example, when the control board 232 is triggered, the driving mechanism is controlled to operate almost simultaneously; or, the driving mechanism is controlled to operate after a period of time after the control board 232 is triggered.

The driving mechanism includes a piston 240, a connecting member and a driving motor 242. The piston 240 is located in the liquid storage chamber 220. The piston 240 is similar to the piston of the hydraulic cylinder, and divides the liquid storage chamber 220 into a hollow chamber 220b and a liquid chamber 220a. The hollow chamber 220b does not store the e-cigarette liquid. The liquid chamber 220a is used for storing the e-cigarette liquid, the liquid chamber 220a is in communication with the atomizing chamber 211 through the liquid inlet tube 222. The connecting member is connected between the piston 240 and the driving motor 242. When the piston 240 moves toward the liquid chamber 220a, the volume of the liquid chamber 220a is compressed, the e-cigarette liquid can be squeezed into the liquid inlet tube 222 and enter the atomizing chamber 211 via the liquid inlet tube 222.

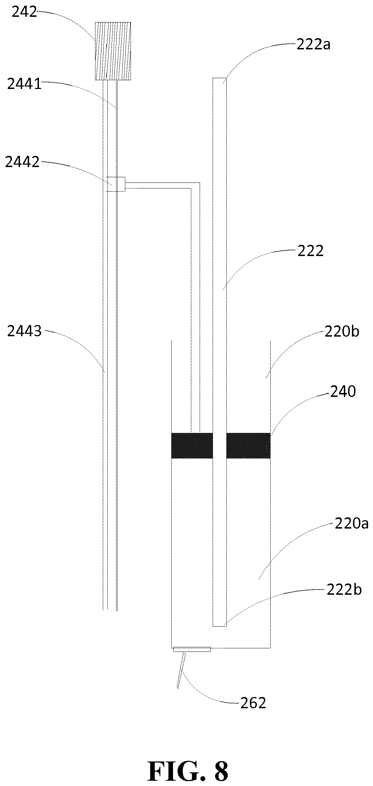

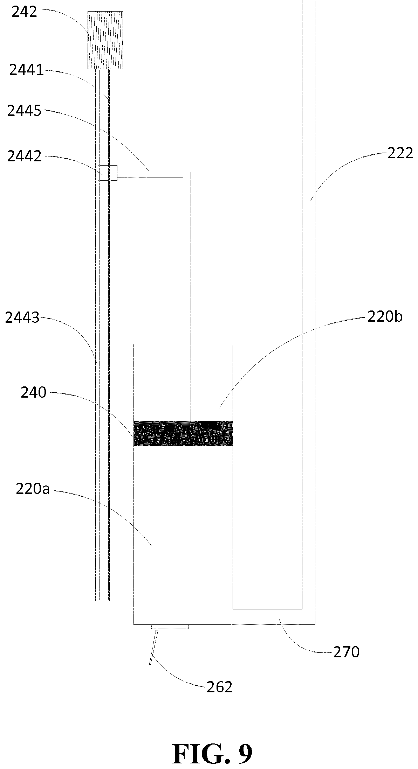

The connecting member is used for converting a rotational motion of the driving motor 242 into a linear motion of the piston 240. Referring to FIG. 8 and FIG. 9, the connecting member includes a screw rod 2441, a slider 2442, a guide rail 2443 and a connecting rod 2445. The screw rod 2441 is connected to the driving motor 242, and the driving motor 242 drives the screw rod 2441 to rotate. The screw rod 2441 extends through the slider 2442, the slider 2442 is slidably connected to the guide rail 2443, such that the rotational motion of the driving motor 242 is converted into the linear motion of the slider 2442. One end of the connecting rod 2445 is fixedly connected to the slider 2442, and the other end of the connecting rod 2445 is fixedly connected to the piston 240. Therefore, when the slider 2442 is linearly moved, the piston 240 can be driven to perform linear motion therewith. With the structure of the screw rod, a precise and quantitative liquid intake can be achieved. One turn rotation of the screw rod 2441 can realize a linear movement of the piston 240 for a certain distance, so that a certain amount of e-cigarette liquid can be supplied. The rotation of the driving motor 242 can be controlled by the control board 232, thereby achieving control the amount of movement of the piston 240, and the quantitative liquid feeding can be realized.

The driving motor 242 of the present disclosure can employ any form of motor. When the driving motor 242 is a stepper motor or a servo motor, the movement of the piston 240 can be more precisely controlled.

As long as the rotational motion of the driving motor 242 can be converted into the linear motion of the piston 240, the connecting member can take any other form. For example, in an embodiment not shown, the connecting member is a gear rack structure, that is, a gear is fixedly connected to an output shaft of the driving motor 242, the upper end of the rack is engaged with the gear, and the lower end of the rack is fixedly connected to the piston 240. The piston 240 moves linearly under the drive of the rack.

The connection position of the liquid inlet tube 222 and the liquid storage chamber 220 is not limited. For example, referring to FIG. 8, in one embodiment, the second end 222b of the liquid inlet tube 222 passes through the hollow chamber 220b and the piston 240 in sequence, the second end 222b of the liquid inlet tube 222 is inserted into the liquid chamber 220a. The e-cigarette liquid in the liquid chamber 220a can be injected into the liquid inlet tube 222 as the piston 240 moves from the hollow chamber 220b toward the liquid chamber 220a. Further, the second end 222b of the liquid inlet tube 222 is located close to the bottom of the liquid chamber 220a, so that the e-cigarette liquid can be injected into the liquid inlet tube 222 even when the amount of e-cigarette liquid in the liquid chamber 220a is small.

Referring to FIG. 9, in another embodiment, the liquid storage device further includes a connecting tube 270 disposed between the liquid chamber 220a and the liquid inlet tube 222. The liquid chamber 220a is in communication with the liquid inlet tube 222 through the connecting tube 270, that is, the liquid inlet tube 222 no longer passes through the hollow chamber 220b, but a through hole is formed in the wall of the liquid chamber 220a, the connection tube 270 is connected to the through hole. Further, the through hole corresponding to the connecting tube 270 is located close to the bottom of the liquid chamber 220a, so that the e-cigarette liquid can be squeezed into the liquid inlet tube 222 even when the amount of e-cigarette liquid in the liquid chamber 220a is small.

Referring to FIG. 5, the electronic cigarette further includes a battery device and a housing 250. The battery device includes a battery 252, the battery 252 is located in the housing 250. In this embodiment, the liquid storage device and the driving mechanism are both disposed in the housing 250. That is, the liquid storage device, the driving mechanism and the battery 252 share the same housing 250, the atomizing device 210 is disposed above the housing 250, such that after the atomizing device 210 is connected to the housing 250, the connection between the atomizing device 210, the battery device and the liquid storage device is completed. In another embodiment (not shown), the atomizing device 210, the liquid storage device and the driving mechanism are all disposed within the housing 250.

In order to avoid liquid leakage, a sealing ring is provided at the position where the piston 240 is in contact with the inside wall of the liquid storage chamber 220, that is, the piston 240 and the liquid storage chamber 220 are sealed through the sealing ring. Therefore, the upper end of the hollow chamber 220b can be either closed or opened.

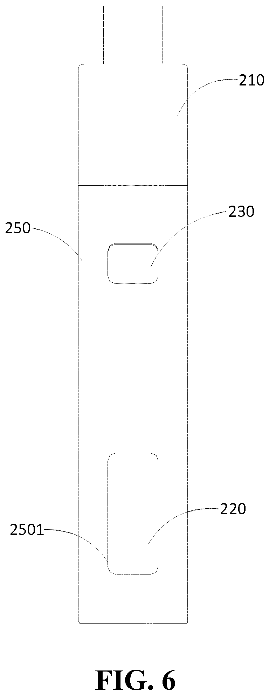

Referring to FIG. 6, in order to facilitate the observation of the amount of e-cigarette liquid in the liquid storage chamber 220, the peripheral wall of the liquid storage chamber 220 can be made of a transparent material such as glass, transparent resin, etc., and an observation window 2501 is formed on the housing 250. The amount of e-cigarette liquid in the liquid storage chamber 220 can be visually observed through the observation window 2501. It can be understood that, the material of the peripheral wall of the liquid storage chamber 220 can also be selected as a non-transparent material. In this case, a liquid level detection device can be disposed in the liquid storage chamber 220, and the electronic cigarette is provided with a display screen correspondingly. The liquid level detection device is electrically connected to the display screen for transmitting the liquid level information detected by the liquid level detection device to the user through the display screen, thereby obtaining the amount of the e-cigarette liquid through the liquid level detection device. It can be understood that, when the electronic cigarette adopts the display touch screen, the display touch screen is the display screen.

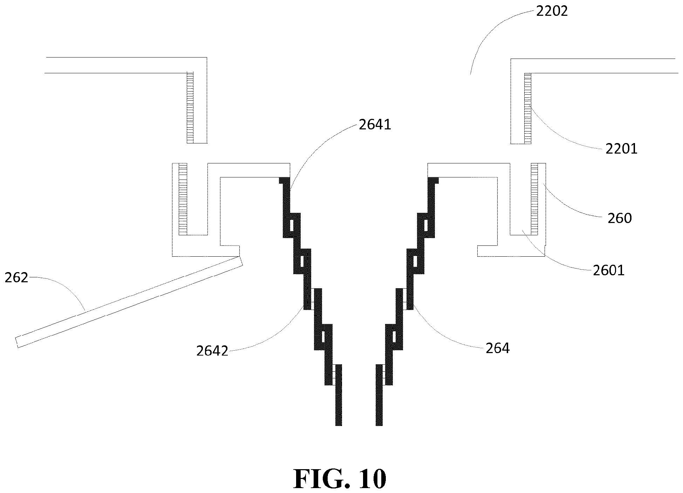

Referring to FIG. 10, an injection hole 2202 is defined at the bottom of the liquid storage chamber 220. An opening is provided at the bottom wall of the housing 250 corresponding to the injection hole 2202, so that the liquid storage chamber 220 can be replenished with the e-cigarette liquid through the opening and the injection hole 2202. The electronic cigarette further includes a liquid injection assembly for covering the opening. The liquid injection assembly includes a mounting member 260, a cover 262, and a telescopic liquid injection tube 264. The telescopic liquid injection tube 264 can be extended or retracted, the telescopic liquid injection tube 264 is fixedly connected to the mounting member 260, the cover 262 is rotatably connected to the mounting member 260. The mounting member 260 is connected to the bottom wall of the housing 250, that is, the liquid injection assembly is connected to the bottom wall of the housing 250 through the mounting member 260. The upper port of the telescopic liquid injection tube 264 is sealing connected to the opening. The cover 262 includes an open state and a closed state. When the cover 262 is in the closed state, the lower port of the telescopic liquid injection tube 264 is sealing abutted against the cover 262. That is, when the cover 262 is in the closed state, the cover 262 seals the lower port of the telescopic liquid injection tube 264, thereby sealing the liquid storage chamber 220, so that the e-cigarette liquid in the liquid storage chamber 220 does not leak through the injection hole 2202. In order to facilitate the control of the liquid injection process, the controller further includes a liquid injection control switch (not shown), by pressing the liquid injection control switch, the electronic cigarette can start to inject liquid. It can be understood that, the liquid injection control switch can be a mechanical physical button or a virtual button on the touch screen. Further, to facilitate the control of the amount of liquid injection, the controller further includes a liquid injection amount control switch for setting and controlling the amount of liquid injection each time. For example, when the electronic cigarette adopts the liquid injection amount control switch of a mechanical type, a plurality of gear positions can be set, each gear position corresponds to the amount of liquid injection each time. When the electronic cigarette adopts the liquid injection amount control switch of a touch screen type, a specific liquid injection amount value can be set on the touch screen with more flexibility.

The liquid injection control switch and the liquid inlet control switch can be separate switches or a common switch. When the liquid injection control switch and the liquid inlet control switch are a common switch, the liquid inlet mode and the liquid injection mode can be switched by the chip in the control board.

The telescopic liquid injection tube 264 includes a first tube 2641 and multiple telescopic tubes 2642. The first tube 2641 is fixedly connected to the mounting member 260, every two adjacent telescopic tubes 2642 are nested and connected, so that a lower telescopic tube 2642 is not separated from an upper telescopic tube 2642. The structure of the telescopic liquid injection tube 264 is similar to that of the engineering crane, and will not be described herein. When opening the cover 262 (i.e., the cover 262 is in the open state), each telescopic tube 2642 is automatically extended under the action of its own gravity. At this time, the telescopic liquid injection tube 264 can be inserted into a liquid bottle, and then the driving motor 242 is controlled to rotate reversely by the controller to drive the piston 240 to move toward the hollow chamber 220b (i.e., the piston 240 moves upward) to achieve automatic liquid injection. When the automatic liquid injection is required, the cover 262 is operated to be in the open state, the telescopic liquid injection tube 264 is extended by gravity, the lower end of the telescopic liquid injection tube 264 is inserted into the liquid bottle. At this time, by clicking the liquid injection control switch or the liquid inlet control switch (when the liquid inlet control switch and the liquid injection control switch share the same switch), the automatic liquid injection is started. In addition, the amount of rotation of the driving motor 242 can further be set in the controller, thereby controlling the movement stroke of the piston 240 to achieve quantitative liquid injection. Of course, e-cigarette liquid can be directly poured into the liquid storage chamber 220 after opening the bottom cover and inverting the electronic cigarette, but this cannot accurately control the injection amount. Further, a one-way conducting member (not shown) is disposed in the liquid inlet tube 222, the one-way conducting member allows the e-cigarette liquid in the liquid storage chamber 220 to enter the atomizing chamber 211 in one-way manner. It can be understood that, the one-way conducting member can also be disposed in the connecting tube 270. The one-way conducting member prevents the e-cigarette liquid in the atomizing chamber 211 from flowing back into the liquid storage chamber 220 when the e-cigarette liquid is injected into the liquid storage chamber 220 through the liquid injection assembly. It can be understood that, the one-way conducting member can be a one-way valve or a one-way membrane.

Furthermore, a convex ring 2201 protrudes outward from the bottom of the liquid storage chamber 220 around the injection hole 2202. The mounting member 260 is provided with a groove 2601 for engaging with the convex ring 2201. When the convex ring 2201 is embedded in the groove 2601, the liquid storage chamber 220 and the mounting member 260 are detachably connected by the convex ring 2201 and the groove 2601. There are many ways to achieve detachable connection, such as snap connection and threaded connection. For example, in the embodiment, a threaded connection is used. Specifically, referring to FIG. 10, the convex ring 2201 is provided with an external thread, the groove 2601 is provided with an internal thread, the convex ring 2201 is screwed connected to the groove 2601. The center of the mounting member 260 has a through hole corresponding to the injection hole 2202. The upper end of the telescopic liquid injection tube 264 is fixedly connected to the mounting member 260, the upper port of the telescopic liquid injection tube 264 corresponds to the through hole of the mounting member 260. The telescopic liquid injection tube 264 communicates with the liquid storage chamber 220 through the through hole.

Various other changes and modifications may be made by those skilled in the art in light of the above-described technical solutions and concepts, and all such changes and modifications are intended to fall within the scope of the appended claims.

## Claims

1. An electronic cigarette, comprising a liquid storage device (120) and an atomizing device (110), the liquid storage device (120) comprising a liquid storage chamber (121), the atomizing device (110) comprising an atomizing chamber (111), wherein the electronic cigarette further comprises a controller (140) and a driving mechanism (150), the driving mechanism (150) comprises a driving motor (151), a screw rod (152) and a slider (153), the screw rod (152) is connected to the driving motor (151), the slider (153) is connected to the screw rod (152), the driving motor (151) is electrically connected to and controlled by the controller (140); **characterized in that** when the controller (140) controls a liquid intake of the electronic cigarette during use of the electronic cigarette, the controller (140) controls the driving motor (151) to drive the screw rod (152), the screw rod (152) then drives the slider (153) to move in a direction close to the liquid storage chamber (121) so as to drive e-cigarette liquid in the liquid storage chamber (121) to enter the atomizing chamber (111); after use of the electronic cigarette, the controller (140) controls the driving motor (151) to drive the screw rod (152), the screw rod (152) then drives the slider (153) to move in a direction away from the liquid storage chamber (121) so as to drive residual e-cigarette liquid in the atomizing chamber (111) to return back to the liquid storage chamber (121).

2. The electronic cigarette according to claim **1,** wherein the electronic cigarette further comprises a guiding groove (1321) in communication with the liquid storage chamber (121), the atomizing device (110) further comprises a liquid inlet (112), the atomizing chamber (111) is in communication with the liquid storage chamber (121) through the liquid inlet (112), the screw rod (152) and the slider (153) are disposed in the guiding groove (1321), the driving motor (151) is disposed at a side of the guiding groove (1321) away from the liquid storage chamber (121).

3. The electronic cigarette according to claim **2,** wherein the slider (153) is slidably connected to the screw rod (152); when the driving motor (151) rotates, the screw rod (152) is driven to rotate and drives the slider (153) to slide in the direction close to the liquid storage chamber (121) or to slide in the direction away from the liquid storage chamber (121); or the slider (153) is fixed at one end of the screw rod (152) near the liquid storage chamber (121), the screw rod (152) is driven by the driving motor (151) to make a telescopic motion and drives the slider (153) to move in the direction close to the liquid storage chamber (121) or in the direction away from the liquid storage chamber (121) in the guiding groove (1321).

4. The electronic cigarette according to claim **2,** wherein the controller (140) comprises a control switch (141) and a control board (142), the control switch (141) is signally connected to the control board (142), the driving motor (151) is electrically connected to and controlled by the control board (142); the controller (140) further comprises an input device (143), the input device (143) is signally connected to the control board (142), the input device (143) is configured for inputting desired operating temperature/operating power/operating voltage of the atomizing device (110) to the control board (142); the control switch (141) is a key switch or a touch switch, the control board (142) is preset with a corresponding relationship between the rotational speed of the driving motor (151) and the operating temperature/operating power/operating voltage of the atomizing device (110).

5. The electronic cigarette according to claim **2,** wherein the controller (140) comprises a control switch (141) and a control board (142), the control switch (141) is signally connected to the control board (142), the driving motor (151) is electrically connected to and controlled by the control board (142), the control switch (141) is an airflow sensor configured to detect the flow velocity of the airflow when the user is smoking, the control board (142) is preset with a corresponding relationship between the rotational speed of the driving motor (151) and the flow velocity of the airflow.

6. An electronic cigarette, comprising a liquid storage device and an atomizing device (210), the liquid storage device comprising a liquid storage chamber (220), the atomizing device (210) comprising an atomizing chamber (211), wherein the electronic cigarette further comprises a controller and a driving mechanism, the driving mechanism comprises a piston (240), a connecting member and a driving motor (242), the piston (240) is located in the liquid storage chamber (220) and the piston (240) divides the liquid storage chamber (220) into a hollow chamber (220b) and a liquid chamber (220a), the connecting member is connected between the piston (240) and the driving motor (242), the driving motor (242) is electrically connected to and controlled by the controller; **characterized in that** when the controller controls a liquid intake of the electronic cigarette during use of the electronic cigarette, the controller controls the driving motor (242) to drive the connecting member, the connecting member then drives the piston (240) to move towards the liquid chamber (220a) so as to drive e-cigarette liquid in the liquid storage chamber (220) to enter the atomizing chamber (211); the electronic cigarette further comprises a housing (250), the liquid storage device is located in the housing (250), an injection hole (2202) is defined at a bottom of the liquid storage chamber (220), an opening is provided at the housing (250) corresponding to the injection hole (2202), the electronic cigarette further comprises a liquid injection assembly for covering the opening; when injection of e-cigarette liquid is required and the injection hole (2202) is in communication with a liquid bottle, the drive motor (242) is controlled by the controller to rotate reversely, leading the connecting member to drive the piston (240) to move towards the hollow chamber (220b) so as to achieve automatic liquid injection into the liquid chamber (220a).

7. The electronic cigarette according to claim **6,** wherein the liquid storage device further comprises a liquid inlet tube (222), a first end (222a) of the liquid inlet tube (222) is in communication with the atomizing chamber (211), a second end (222b) of the liquid inlet tube (222) is in communication with the liquid chamber (220a), the controller controls the driving motor (242) to drive the e-cigarette liquid in the liquid chamber (220a) into the atomizing chamber (211) through the liquid inlet tube (222).

8. The electronic cigarette according to claim **7,** wherein the second end (222b) of the liquid inlet tube (222) passes through the hollow chamber (220b) and the piston (240) in sequence, and the second end (222b) of the liquid inlet tube (222) is inserted into the liquid chamber (220a).

9. The electronic cigarette according to claim **6,** wherein the connecting member comprises a screw rod (2441), a slider (2442), a guide rail (2443) and a connecting rod (2445), the screw rod (2441) is connected to the driving motor (242), the screw rod (2441) extends through the slider (2442), the slider (2442) is slidably connected to the guide rail (2443), one end of the connecting rod (2445) is fixedly connected to the slider (2442), and the other end of the connecting rod (2445) is fixedly connected to the piston (240).

10. The electronic cigarette according to claim **6,** wherein the liquid injection assembly comprises a mounting member (260), a cover (262) and a telescopic liquid injection tube (264), the telescopic liquid injection tube (264) is fixedly connected to the mounting member (260), the cover (262) is rotatably connected to the mounting member (260), the mounting member (260) is connected to the housing (250), an upper port of the telescopic liquid injection tube (264) is sealing connected to the opening, the cover (262) has an open state and a closed state, when the cover (262) is in the closed state, a lower port of the telescopic liquid injection tube (264) is sealing abutted against the cover (262).

11. The electronic cigarette according to claim **10,** wherein the telescopic liquid injection tube (264) comprises a first tube (2641) and a plurality of telescopic tubes (2642), the first tube (2641) is fixedly connected to the mounting member (260), every two adjacent telescopic tubes (2642) are nested and connected.

12. The electronic cigarette according to claim **7,** wherein a one-way conducting member is located in the liquid inlet tube (222), the one-way conducting member prevents the e-cigarette liquid in the atomizing chamber (211) from flowing back into the liquid storage chamber (220) when the e-cigarette liquid is injected into the liquid storage chamber (220) through the liquid injection assembly.

## Patentansprüche

1. Eine elektronische Zigarette, die eine Flüssigkeitsspeichervorrichtung (120) und eine Zerstäubungsvorrichtung (110) umfasst, die Flüssigkeitsspeichervorrichtung (120), die eine Flüssigkeitsspeicherkammer (121) umfasst, die Zerstäubungsvorrichtung (110), die eine Zerstäubungskammer (111) umfasst, wobei die elektronische Zigarette ferner einen Regler (140) und einen Antriebsmechanismus (150) umfasst, der Antriebsmechanismus (150) einen Antriebsmotor (151), eine Gewindestange (152) und einen Schieber (153) umfasst, die Gewindestange (152) mit dem Antriebsmotor (151) verbunden ist, der Schieber (153) mit der Gewindestange (152) verbunden ist, der Antriebsmotor (151) elektrisch mit dem Regler (140) verbunden ist und von diesem gesteuert wird; **dadurch gekennzeichnet, dass**, wenn der Regler (140) während des Gebrauchs der elektronischen Zigarette die Flüssigkeitsaufnahme der elektronischen Zigarette steuert, der Regler (140) den Antriebsmotor (151) steuert, um die Gewindestange (152) anzutreiben, die Gewindestange (152) treibt dann den Schieber (153) an, um ihn in die Richtung der Flüssigkeitsspeicherkammer (121) zu bewegen, damit die E-Zigarettenflüssigkeit in die Flüssigkeitsspeicherkammer (121) getrieben wird, um in die Zerstäubungskammer (111) einzufließen; nach Verwendung der elektronischen Zigarette steuert der Regler (140) den Antriebsmotor (151), um die Gewindestange (152) anzutreiben, die Gewindestange (152) treibt dann den Schieber (153) an, um ihn in die Richtung weg von der Flüssigkeitsspeicherkammer (121) zu bewegen, damit die restliche E-Zigarettenflüssigkeit in die Zerstäubungskammer (111) getrieben wird, um zur Flüssigkeitsspeicherkammer (121) zurückzufließen.

2. Elektronische Zigarette nach Anspruch 1, wobei die elektronische Zigarette ferner eine Führungsnut (1321) in Verbindung mit der Flüssigkeitsspeicherkammer (121) umfasst, die Zerstäubungsvorrichtung (110) ferner einen Flüssigkeitseinlass (112) umfasst, die Zerstäubungskammer (111) mit der Flüssigkeitsspeicherkammer (121) über den Flüssigkeitseinlass (112) in Verbindung steht, die Gewindestange (152) und der Schieber (153) in der Führungsnut (1321) angeordnet sind, der Antriebsmotor (151) an der Seite der Führungsnut (1321) mit Abstand von der Flüssigkeitsspeicherkammer (121) angeordnet ist.

3. Elektronische Zigarette nach Anspruch 2, wobei der Schieber (153) verschiebbar mit der Gewindestange (152) verbunden ist; wenn sich der Antriebsmotor (151) dreht, wird die Gewindestange (152) angetrieben, um sich zu drehen und den Schieber (153) in die Richtung der Flüssigkeitsspeicherkammer (121) oder in die Richtung weg von der Flüssigkeitsspeicherkammer (121) anzutreiben; oder der Schieber (153) ist an einem Ende der Gewindestange (152) in der Nähe der Flüssigkeitsspeicherkammer (121) befestigt, die Gewindestange (152) wird vom Antriebsmotor (151) angetrieben, um eine Teleskopbewegung auszuführen, und treibt den Schieber (153) an, um sich in die Richtung der Flüssigkeitsspeicherkammer (121) oder in die Richtung weg von der Flüssigkeitsspeicherkammer (121) in der Führungsnut (1321) zu bewegen.

4. Elektronische Zigarette nach Anspruch 2, wobei der Regler (140) einen Reglerschalter (141) und eine Reglerplatine (142) umfasst, der Reglerschalter (141) mit der Reglerplatine (142) Signale austauscht und der Antriebsmotor (151) elektrisch mit der Reglerplatine (142) verbunden ist und von dieser gesteuert wird; der Regler (140) umfasst ferner ein Eingabegerät (143), das Eingabegerät (143) tauscht mit der Reglerplatine (142) Signale aus, das Eingabegerät (143) ist für die Eingabe der gewünschten Betriebstemperatur/Betriebsleistung/Betriebsspannung der Zerstäubungsvorrichtung (110) in die Reglerplatine (142) konfiguriert; der Reglerschalter (141) ist ein Schlüsselschalter oder ein Berührungsschalter, die Reglerplatine (142) ist so voreingestellt, dass ein entsprechendes Verhältnis zwischen der Drehzahl des Antriebsmotors (151) und der Betriebstemperatur / Betriebsleistung / Betriebsspannung der Zerstäubungsvorrichtung (110) besteht.

5. Elektronische Zigarette nach Anspruch 2, wobei der Regler (140) einen Reglerschalter (141) und eine Reglerplatine (142) umfasst, der Reglerschalter (141) mit der Reglerplatine (142) Signale austauscht, der Antriebsmotor (151) elektrisch mit der Reglerplatine (142) verbunden ist und von dieser gesteuert wird, der Reglerschalter (141) ein Luftströmungssensor ist, der so konfiguriert ist, dass er die Strömungsgeschwindigkeit des Luftstroms, während der Benutzer raucht, erfasst, und die Reglerplatine (142) so voreingestellt ist, dass ein entsprechendes Verhältnis zwischen der Drehzahl des Antriebsmotors (151) und der Strömungsgeschwindigkeit des Luftstroms besteht.

6. Elektronische Zigarette, die eine Flüssigkeitsspeichervorrichtung und eine Zerstäubungsvorrichtung (210) umfasst, wobei die Flüssigkeitsspeichervorrichtung eine Flüssigkeitsspeicherkammer (220) umfasst, wobei die Zerstäubungsvorrichtung (210) eine Zerstäubungskammer (211) umfasst, wobei die elektronische Zigarette ferner einen Regler und einen Antriebsmechanismus umfasst, wobei der Antriebsmechanismus einen Kolben (240), ein Verbindungselement und einen Antriebsmotor (242) umfasst, wobei der Kolben (240) sich in der Flüssigkeitsspeicherkammer (220) befindet und der Kolben (240) die Flüssigkeitsspeicherkammer (220) in eine Hohlkammer (220b) und eine Flüssigkeitskammer (220a) unterteilt, das Verbindungselement den Kolben (240) mit dem Antriebsmotor (242) verbindet, der Antriebsmotor (242) elektrisch mit dem Regler verbunden ist und von dem gesteuert wird; **dadurch gekennzeichnet, dass**, wenn der Regler eine Flüssigkeitsaufnahme der elektronischen Zigarette während der Verwendung der elektronischen Zigarette steuert, der Regler den Antriebsmotor (242) steuert, um das Verbindungselement anzutreiben, das Verbindungselement dann den Kolben (240) antreibt, um sich in Richtung der Flüssigkeitskammer (220a) zu bewegen, um E-Zigarettenflüssigkeit in die Flüssigkeitsspeicherkammer (220) zu treiben, so dass sie in die Zerstäubungskammer (211) einfließt; die elektronische Zigarette umfasst ferner ein Gehäuse (250), die Flüssigkeitsspeichervorrichtung ist in dem Gehäuse (250) angeordnet, eine Einspritzöffnung (2202) ist am Boden der Flüssigkeitsspeicherkammer (220) angeordnet, eine Öffnung ist an der Einspritzöffnung (2202) des entsprechenden Gehäuse (250) angeordnet, die elektronische Zigarette umfasst ferner eine Flüssigkeitseinspritzvorrichtung um die Öffnung abzudecken; wenn das Einspritzen von E-Zigarettenflüssigkeit erforderlich ist und die Einspritzöffnung (2202) mit der Flüssigkeitsflasche in Verbindung steht, wird der Antriebsmotor (242) von dem Regler so gesteuert, dass er sich umgekehrt dreht, wodurch das Verbindungselement den Kolben (240) antreibt, um sich in Richtung der Hohlkammer (220b) zu bewegen, so dass das Flüssigkeitseinspritzen in die Flüssigkeitskammer (220a) automatisch erzeugt wird.

7. Elektronische Zigarette nach Anspruch 6, wobei der Flüssigkeitsspeicher ferner ein Flüssigkeitseinlassrohr (222) umfasst, wobei das erste Ende (222a) des Flüssigkeitseinlassrohrs (222) mit der Zerstäubungskammer (211) in Verbindung steht, und das zweite Ende (222b) des Flüssigkeitseinlassrohrs (222) mit der Flüssigkeitskammer (220a) in Verbindung steht; der Regler steuert den Antriebsmotor (242), um die E-Zigarettenflüssigkeit in der Flüssigkeitskammer (220a) in die Zerstäubungskammer (211) durch das Flüssigkeitseinlassrohr (222) zu treiben.

8. Elektronische Zigarette nach Anspruch 7, wobei das zweite Ende (222b) des Flüssigkeitseinlassrohrs (222) nacheinander durch die Hohlkammer (220b) und den Kolben (240) verläuft, und das zweite Ende (222b) des Flüssigkeitseinlassrohrs (222) in die Flüssigkeitskammer (220a) eingeführt ist.

9. Elektronische Zigarette nach Anspruch 6, wobei das Verbindungselement eine Gewindestange (2441), einen Schieber (2442), eine Führungsschiene (2443) und eine Verbindungsstange (2445) umfasst, wobei die Gewindestange (2441) mit dem Antriebsmotor (242) verbunden ist, die Gewindestange (2441) sich durch den Schieber (2442) erstreckt, der Schieber (2442) mit der Führungsschiene (2443) verschiebbar verbunden ist, ein Ende der Verbindungsstange (2445) fest mit dem Schieber (2442) verbunden ist und das andere Ende der Verbindungsstange (2445) fest mit dem Kolben (240) verbunden ist.

10. Elektronische Zigarette nach Anspruch 6, wobei die Flüssigkeitseinspritzvorrichtung ein Befestigungselement (260), eine Abdeckung (262) und ein Flüssigkeitseinspritzteleskoprohr (264) umfasst, wobei das Flüssigkeitseinspritzteleskoprohr (264) fest mit dem Befestigungselement (260) verbunden ist, die Abdeckung (262) drehbar mit dem Befestigungselement (260) verbunden ist, das Befestigungselement (260) mit dem Gehäuse (250) verbunden ist, der obere Anschluss des Flüssigkeitseinspritzteleskoprohrs (264) mit der Öffnung abdichtend verbunden ist, die Abdeckung (262) einen offenen Zustand und einen geschlossenen Zustand aufweist; wenn die Abdeckung (262) sich im geschlossenen Zustand befindet, ist der untere Anschluss des Flüssigkeitseinspritzteleskoprohrs (264) abdichtend mit der Abdeckung (262) verbunden.

11. Elektronische Zigarette nach Anspruch 10, wobei das Flüssigkeitseinspritzteleskoprohr (264) ein erstes Rohr (2641) und mehrere Teleskoprohre (2642) umfasst, wobei das erste Rohr (2641) fest mit dem Befestigungselement (260) verbunden ist, und alle zwei benachbarten Teleskoprohre (2642) verschachtelt und verbunden sind.

12. Elektronische Zigarette nach Anspruch 7, wobei sich ein Einweg-Leitelement in dem Flüssigkeitseinlassrohr (222) befindet, wobei das Einweg-Leitelement verhindert, dass die E-Zigarettenflüssigkeit der Zerstäubungskammer (211) in die Flüssigkeitsspeicherkammer (220) zurückfließt, wenn die E-Zigarettenflüssigkeit durch die Flüssigkeitseinspritzvorrichtung in die Flüssigkeitsspeicherkammer (220) eingespritzt wird.

## Revendications

1. Une cigarette électronique comprenant un dispositif de stockage de liquide (120) et un dipositif de pulvérisation (110), le dispositif de stockage de liquide (120) comprend un compartiment de stockage de liquide (121), le dispositif de pulvérisation (110) comprend un compartiment de pulvérisation (111), dans lequel la cigarette électronique comprend, en outre, un dispositif de commande (140) et un mécanisme d'entraînement (150), ledit mécanisme d'entraînement (150) comprend un moteur d'entraînement (151), une tige filetée (152) et un curseur (153), la tige filetée (152) est raccordée au moteur d'entrainement (151), le curseur (153) est raccordé à la tige filetée (152), le moteur d'entraînement (151) est électriquement couplé à un régulateur (140) dont il est commandé ; **caractérisée en ce que**, lorsque le régulateur (140) commande une arrivée de liquide de la cigarette électronique au cours de l'utilisation de ladite cigarette électronique, le régulateur (140) commande au moteur d'entraînement (151) d'entraîner la tige filetée (152), ce qui amène la tige filetée (152) à entraîner le curseur (153) à coulisser en direction du compartiment de stockage de liquide (121) de manière à entraîner une quantité du liquide contenu dans le compartiment de stockage de liquide (121) à pénétrer dans le compartiment de pulvérisation (111) ; après une utilisation de la cigarette électronique, le régulateur (140) commande au moteur d'entraînement (151) d'entraîner la tige filetée (152), ladite tige filetée (152) entraîne le curseur (153) à coulisser dans une direction qui l'éloigne du compartiment de stockage de liquide (121) de manière à entraîner le liquide résiduel de la cigarette électronique contenu dans le compartiment de pulvérisation (111) à retourner dans le compartiment de stockage de liquide (121).

2. La cigarette électronique correspondant à la revendication **1,** dans laquelle la cigarette électronique comprend, en outre, une rainure de guidage (1321) reliée au compartiment de stockage de liquide (121), le dispositif de pulvérisation (HO) comprend, en outre, une arrivée de liquide (112), le compartiment de pulvérisation (111) est relié au compartiment de stockage de liquide (121) par le biais de l'arrivée de liquide (112), la tige filetée (152) et le curseur (153) sont placés dans la rainure de guidage (1321), le moteur d'entraînement (151) est placé sur le côté de la rainure de guidage (1321) qui est éloigné du compartiment de stockage de liquide (121).

3. La cigarette électronique correspondant à la revendication **2,** dans laquelle le curseur (153) coulisse en liaison avec la tige filetée (152) ; lorsque le moteur d'entraînement (151) est en rotation, la tige filetée (152) est amenée à tourner en entraînant ainsi le curseur (153) à coulisser en direction de rapprochement avec le compartiment de stockage de liquide (121) ou à coulisser dans la direction qui l'éloigne du compartiment de stockage de liquide (121) ; ou alors le curseur (153) est fixé à l'une des extrémités de la tige filetée (152) à proximité du compartiment de stockage de liquide (121), la tige filetée (152) est entraînée par le moteur d'entraînement (151) de manière à effectuer un mouvement de télescope et à entraîner le curseur (153) à coulisser dans une direction de rapprochement avec le compartiment de stockage de liquide (121) suivant la rainure de guidage (1321).

4. La cigarette électronique correspondant à la revendication **2,** dans laquelle le régulateur (140) comprend un commutateur de commande (141) et une carte de commande (142), le commutateur de commande (141) est relié à la carte de commande (142) par voie d'émission d'impulsions, le moteur d'entraînement (151) est électriquement branché à la carte de commande (142) dont il est commandé ; le régulateur (140) comprend en outre un dispositif d'entrée (143), ledit dispositif d'entrée (143) est relié, par voie d'émission d'impulsions, à la carte de commande (142), le dispositif d'entrée (143) est configuré pour l'entrée du paramétrage souhaité en termes de température de fonctionnement/puissance de fonctionnement/tension de fonctionnement du dispositif de pulvérisation (110) à destination de la carte de commande (142) ; le commutateur de commande (141) est sous forme de touche ou de commutateur tactile, la carte de commande (142) est préréglée sur un lien correspondant entre la vitesse de rotation du moteur d'entraînement (151) et la température de fonctionnement/la puissance de fonctionnement/la tension de fonctionnement du dispositif de pulvérisation (110).

5. La cigarette électronique correspondant à la revendication **2,** dans laquelle le régulateur (140) comprend un commutateur de commande (141) et une carte de commande (142), le commutateur de commande (141) est relié, par voie d'impulsions, à la carte de commande (142), le moteur d'entraînement (151) est électriquement branché à la carte de commande (142) dont il est commandé, le commutateur de commande (141) est un capteur de flux d'air configuré de manière à détecter la vitesse de débit du flux d'air, lorsque l'utilisateur est en train de fumer, la carte de commande (142) est préréglée sur un lien correspondant entre la vitesse de rotation du moteur d'entraînement (151) et la vitesse de débit du flux d'air.

6. Une cigarette électronique comprenant un dispositif de stockage de liquide et un dispositif de pulvérisation (210), un dispositif de stockage de liquide comprenant un compartiment de stockage de liquide (220), un dispositif de pulvérisation (210) comprenant un compartiment de pulvérisation (211), dans laquelle la cigarette électronique comprend, en outre, un régulateur et un mécanisme d'entraînement, ledit mécanisme d'entraînement comprend un piston (240), une articulation de raccordement et un moteur d'entraînement (242), le piston est placé dans le compartiment de stockage de liquide (220) et le piston (240) divise le compartiment de stockage (220) en un compartiment creux (220b) et un compartiment contenant le liquide (220a), l'articulation de raccordement est branchée entre le piston (240) et le moteur d'entraînement (242), ledit moteur d'entraînement (242) est électriquement branché au régulateur dont il est commandé, **caractérisé en ce que**, lorsque le régulateur commande une arrivée de liquide de la cigarette électronique au cours de l'utilisation de la cigarette électronique, le régulateur commande au moteur d'entraînement (242) d'entrainer l'articulation de raccordement, ladite articulation de raccordement entraîne alors le piston (240) à se déplacer en direction du compartiment de liquide (220a) de manière à amener du liquide contenu dans le compartiment de stockage de liquide (220) à pénétrer dans le compartiment de pulvérisation (211) ; la cigarette électronique comprend, en outre, un boitier (250), qui abrite le dispositif de stockage de liquide, un orifice d'injection (2202) est délimité sur le fond du compartiment de stockage de liquide (220), une ouverture est aménagée dans le boîtier (250) correspondant à l'orifice d'injection (2202), la cigarette électronique comprend, en outre, un ensemble d'injection pour recouvrir l'ouverture, ; lorsque une injection de liquide de cigarette électronique est nécessaire et l'orifice d'injection (2202) est en communication avec une bouteille de liquide, le moteur d'entraînement (242) est amené par le régulateur à tourner en rotation inversée, conduisant l'articulation de raccordement à entraîner le piston (240) à se mouvoir en direction du compartiment creux (220b) de manière à effectuer une injection de liquide automatique dans le compartiment de liquide (220a).

7. La cigarette électronique correspondant à la revendication **6,** dans laquelle le dispositif de stockage de liquide comprend, en outre, un tuyau d'arrivée de liquide (222), une première extrémité (222a) du tuyau d'arrivée de liquide (222) communique avec le compartiment de pulvérisation (211), une seconde extrémité (222b) du tuyau d'arrivée de liquide (222) communique avec le compartiment du liquide (220a), le régulateur commande au moteur d'entraînement (242) de conduire le liquide de la cigarette électronique contenu dans le compartiment de liquide (220a) dans le compartiment de pulvérisation (211) par le biais du tuyau d'arrivée de liquide (222).

8. La cigarette électronique correspondant à la revendication **7** dans laquelle la seconde extrémité (222b) du tuyau d'arrivée de liquide (222) passe successivement par le compartiment creux (220b) et le piston (240) et la seconde extrémité (222b) du tuyau d'arrivée de liquide (222) est introduite dans le compartiment de liquide (220a).

9. La cigarette électronique correspondant à la revendication 6, dans laquelle l'articulation de raccordement comprend une tige filetée (2441), un curseur (2442), un rail de guidage (2443) et une tige de raccordement (2445), la tige filetée (2441) est reliée au moteur d'entraînement (242), la tige filetée (2441) prolonge le curseur (2442), le curseur (2442) coulisse sur le rail de guidage (2443), une des extrémités de la tige de raccordement (2445) est raccordée de façon fixe au curseur (2442), et l'autre extrémité de la tige de raccordement (2245) est raccordée de façon fixe au piston (240).

10. La cigarette électronique correspondant à la revendication **6,** dans laquelle l'ensemble d'injection de liquide comprend un élément de montage (260), un couvercle (262) et un tuyau télescopique d'injection de liquide (264) est raccordé de façon fixe à l'élément de montage (260), le couvercle (262) est raccordé de manière rotative à l'élément de montage (260), ledit élément de montage (260) est raccordé au boîtier (250), un port supérieur du tuyau télescopique d'injection de liquide (264) assure l'étanchéité, raccordé à l'ouverture, le couvercle (262) dispose d'un état d'ouverture et d'un état de fermeture, lorsque le couvercle (262) est en état de fermeture, un port inférieur du tuyau télescopique d'injection de liquide (264) assure l'étanchéité, placé en butée contre le couvercle (262).

11. La cigarette électronique correspondant à la revendication **10,** dans laquelle le tuyau télescopique (264) comprend un premier tuyau (2641) et une multitude de tuyaux télescopiques (2642), le premier tuyau (2641) est raccordé de façon fixe à l'élément de montage (260), les tuyaux télescopiques adjacents sont emboîtés et raccordés deux par deux.

12. La cigarette électronique correspondant à la revendication **7,** dans laquelle un élément conducteur unidirectionnel est placé dans le tuyau d'arrivée de liquide (222), l'élément conducteur unidirectionnel empêche le liquide de la cigarette électronique contenu dans le compartiment de pulvérisation (211) de refluer dans le compartiment de stockage (220) lorsque le liquide de la cigarette électronique est injecté dans le compartiment de stockage de liquide (220) par le biais de l'ensemble d'injection de liquide.
